# EUROPEAN PATENT APPLICATION

(11) **EP 2 562 267 A2**
(43) Date of publication of application: **27.02.2013**
(21) Application number: 12181589.8
(22) Date of filing: 23.08.2012
(51) Int. Cl.: C12Q 1/68

(54) **Method and kit for identifying Phalaenopsis varieties**

(30) Priority: 24.08.2011 TW 100130368
(71) Applicant: National Cheng Kung University, Tainan City 701 (TW)
(72) Inventor: Wu, Wen-Luan, 701 Tainan City (TW); Kuo, Yi-Tzu, 852 Kaohsiung City (TW); Lee, Yu-Ling, 840 Kaohsiung City (TW); Chung, Yu-Lin, 701 Tainan City (TW)
(74) Representative: Albutt, Jodie

(57) **Abstract**

A method and a kit for identifying Phalaenopsis varieties are disclosed. The kit for identifying Phalaenopsis varieties of the present invention comprises: at least one microsatellite primer pair, wherein each of the microsatellite primer pair comprises: nucleotides with 75-100% identity to the sequences represented by the SEQ ID NOs: 1 to 72.

SEQUENCE LISTING

<110>  National Cheng Kung University

<120>  Method and kit for identifying Phalaenopsis varieties

<130>  S4359_100-118AP

<150>  TW 100130368
<151>  2011-08-24

<160>  72

<170>  PatentIn version 3.3

<210>  1
<211>  22
<212>  DNA
<213>  Artificial

<220>
<223>  Artificial

<400>  1
gcccaatttt cagttttcct tc                                                22


<210>  2
<211>  24
<212>  DNA
<213>  Artificial

<220>
<223>  Artificial

<400>  2
ttgcttcatt gtttttcctt tcac                                              24


<210>  3
<211>  20
<212>  DNA
<213>  Artificial

<220>
<223>  Artificial

<400>  3
tctttgtgtg tgtgtgcgtg                                                   20


<210>  4
<211>  19
<212>  DNA
<213>  Artificial

<220>
<223>  Artificial

<400>  4
gtgtcccgta gacttcccg                                                    19


<210>  5
<211>  18

<212>  DNA
<213>  Artificial

<220>
<223>  Artificial

<400>  5
ggcgagcgat tggtgtgc                                                    18


<210>  6
<211>  22
<212>  DNA
<213>  Artificial

<220>
<223>  Artificial

<400>  6
ttttttcctc attcggtcag gc                                               22


<210>  7
<211>  20
<212>  DNA
<213>  Artificial

<220>
<223>  Artificial

<400>  7
cttgagcgga ttgggtctta                                                  20


<210>  8
<211>  20
<212>  DNA
<213>  Artificial

<220>
<223>  Artificial

<400>  8
cactcagcca acgcatcgaa                                                  20


<210>  9
<211>  21
<212>  DNA
<213>  Artificial

<220>
<223>  Artificial

<400>  9
gcaatttgac taccccctct c                                                21


<210>  10
<211>  22
<212>  DNA
<213>  Artificial

<220>
<223>  Artificial

<400>  10
gcatggatta accgtttttc tt                                                22

<210>  11
<211>  20
<212>  DNA
<213>  Artificial

<220>
<223>  Artificial

<400>  11
ttcggactac aaggcgtacc                                                   20

<210>  12
<211>  21
<212>  DNA
<213>  Artificial

<220>
<223>  Artificial

<400>  12
catttggctc aggctcagta c                                                 21

<210>  13
<211>  20
<212>  DNA
<213>  Artificial

<220>
<223>  Artificial

<400>  13
ctcattcggt caggcatttc                                                   20

<210>  14
<211>  20
<212>  DNA
<213>  Artificial

<220>
<223>  Artificial

<400>  14
agctgcttcc caagtgattc                                                   20

<210>  15
<211>  19
<212>  DNA
<213>  Artificial

<220>
<223>  Artificial

<400>  15
gaatgagggc accaaatct                                                    19

<210>  16
<211>  20
<212>  DNA
<213>  Artificial

<220>
<223>  Artificial

<400>  16
tccattaacc ccgagaaaaa                                                    20


<210>  17
<211>  22
<212>  DNA
<213>  Artificial

<220>
<223>  Artificial

<400>  17
ggaaccaaca agaagaagaa gc                                                 22


<210>  18
<211>  21
<212>  DNA
<213>  Artificial

<220>
<223>  Artificial

<400>  18
gcatcgcaga gaacagagag c                                                  21


<210>  19
<211>  20
<212>  DNA
<213>  Artificial

<220>
<223>  Artificial

<400>  19
gggatggagg gatttgagat                                                    20


<210>  20
<211>  20
<212>  DNA
<213>  Artificial

<220>
<223>  Artificial

<400>  20
aagcacagga aacgcaaact                                                    20


<210>  21
<211>  19
<212>  DNA
<213>  Artificial

<220>
<223>  Artificial

<400>  21
gagcccgatc acaacgacc                                                    19


<210>  22
<211>  20
<212>  DNA
<213>  Artificial

<220>
<223>  Artificial

<400>  22
gtgactccgt tccatgcctc                                                   20


<210>  23
<211>  24
<212>  DNA
<213>  Artificial

<220>
<223>  Artificial

<400>  23
aagatgggta gtatgtgaga atgc                                              24


<210>  24
<211>  20
<212>  DNA
<213>  Artificial

<220>
<223>  Artificial

<400>  24
atcagccaca aagcagaagc                                                   20


<210>  25
<211>  20
<212>  DNA
<213>  Artificial

<220>
<223>  Artificial

<400>  25
ggaagcaggc aaaggtatga                                                   20


<210>  26
<211>  20
<212>  DNA
<213>  Artificial

<220>
<223>  Artificial

<400>  26
ttgctgtctc cctcatctgc                                                   20

<210>  27
<211>  20
<212>  DNA
<213>  Artificial

<220>
<223>  Artificial

<400>  27
agcctacctg attcgccata                                                    20


<210>  28
<211>  20
<212>  DNA
<213>  Artificial

<220>
<223>  Artificial

<400>  28
cccattcatc ccctctctct                                                    20


<210>  29
<211>  20
<212>  DNA
<213>  Artificial

<220>
<223>  Artificial

<400>  29
acactccctg ccaatggtaa                                                    20


<210>  30
<211>  21
<212>  DNA
<213>  Artificial

<220>
<223>  Artificial

<400>  30
tccatttcct tcattttgtg c                                                  21


<210>  31
<211>  20
<212>  DNA
<213>  Artificial

<220>
<223>  Artificial

<400>  31
ggtcagcctc cttcatcaga                                                    20


<210>  32
<211>  21
<212>  DNA

<213>  Artificial

<220>
<223>  Artificial

<400>  32
tggtcatgtg gtgtgttttt a                                                21


<210>  33
<211>  20
<212>  DNA
<213>  Artificial

<220>
<223>  Artificial

<400>  33
tagtggtggt ggcgatgata                                                  20


<210>  34
<211>  23
<212>  DNA
<213>  Artificial

<220>
<223>  Artificial

<400>  34
ctaaataatg ccaaggggta act                                              23


<210>  35
<211>  24
<212>  DNA
<213>  Artificial

<220>
<223>  Artificial

<400>  35
gtaatcacaa ctaaacaggg ctaa                                             24


<210>  36
<211>  24
<212>  DNA
<213>  Artificial

<220>
<223>  Artificial

<400>  36
atgatgatga ggaggagtag attc                                             24


<210>  37
<211>  20
<212>  DNA
<213>  Artificial

<220>
<223>  Artificial

<400>  37
tggtctctgt cgtcacttgg                                                   20


<210>  38
<211>  20
<212>  DNA
<213>  Artificial

<220>
<223>  Artificial

<400>  38
aagaattaca ccgccgatca                                                   20


<210>  39
<211>  21
<212>  DNA
<213>  Artificial

<220>
<223>  Artificial

<400>  39
cattagtggt ctttccctgt g                                                 21


<210>  40
<211>  22
<212>  DNA
<213>  Artificial

<220>
<223>  Artificial

<400>  40
aagagtggat gttttagttt cg                                                22


<210>  41
<211>  20
<212>  DNA
<213>  Artificial

<220>
<223>  Artificial

<400>  41
gctttctgct tatcgttcca                                                   20


<210>  42
<211>  18
<212>  DNA
<213>  Artificial

<220>
<223>  Artificial

<400>  42
gcttacagtg gacgggct                                                     18


<210>  43

<211>  20
<212>  DNA
<213>  Artificial

<220>
<223>  Artificial

<400>  43
tggagggtta gaagcaagga                                                    20


<210>  44
<211>  20
<212>  DNA
<213>  Artificial

<220>
<223>  Artificial

<400>  44
tcatcatccc tcttcctcgt                                                    20


<210>  45
<211>  22
<212>  DNA
<213>  Artificial

<220>
<223>  Artificial

<400>  45
gtttatcttc atgtggtcct cg                                                 22


<210>  46
<211>  20
<212>  DNA
<213>  Artificial

<220>
<223>  Artificial

<400>  46
cttacttgtc cacccccatt                                                    20


<210>  47
<211>  20
<212>  DNA
<213>  Artificial

<220>
<223>  Artificial

<400>  47
ggctcacctt ccttctcctt                                                    20


<210>  48
<211>  22
<212>  DNA
<213>  Artificial

<220>

<223>  Artificial

<400>  48
gctaaaccct aaacccttgt gt                                                22


<210>  49
<211>  20
<212>  DNA
<213>  Artificial

<220>
<223>  Artificial

<400>  49
caactcccaa agcctcaact                                                   20


<210>  50
<211>  20
<212>  DNA
<213>  Artificial

<220>
<223>  Artificial

<400>  50
gacccacacc attccatcac                                                   20


<210>  51
<211>  23
<212>  DNA
<213>  Artificial

<220>
<223>  Artificial

<400>  51
ctaactctct ttccttgctc ctt                                               23


<210>  52
<211>  22
<212>  DNA
<213>  Artificial

<220>
<223>  Artificial

<400>  52
cgtttcttcc tctctcacac tc                                                22


<210>  53
<211>  23
<212>  DNA
<213>  Artificial

<220>
<223>  Artificial

<400>  53
ttggatggta ggtatgagta tgc                                               23

<210>  54
<211>  21
<212>  DNA
<213>  Artificial

<220>
<223>  Artificial

<400>  54
tgattgttct tcccgtctgt t                                                21


<210>  55
<211>  20
<212>  DNA
<213>  Artificial

<220>
<223>  Artificial

<400>  55
cctcgtgctt tcctgttcat                                                  20


<210>  56
<211>  20
<212>  DNA
<213>  Artificial

<220>
<223>  Artificial

<400>  56
agttttgggt gctgctatgc                                                  20


<210>  57
<211>  24
<212>  DNA
<213>  Artificial

<220>
<223>  Artificial

<400>  57
atgaatcgtt tggtatgtat gtga                                             24


<210>  58
<211>  20
<212>  DNA
<213>  Artificial

<220>
<223>  Artificial

<400>  58
attgttcatt gctgctgctg                                                  20


<210>  59
<211>  21
<212>  DNA
<213>  Artificial

<220>
<223>  Artificial

<400>  59
aagttatcca aatgacccca g                                                21


<210>  60
<211>  20
<212>  DNA
<213>  Artificial

<220>
<223>  Artificial

<400>  60
aaacttcggt gcctgagaga                                                  20


<210>  61
<211>  19
<212>  DNA
<213>  Artificial

<220>
<223>  Artificial

<400>  61
ccgctcttcc agtttcgtt                                                   19


<210>  62
<211>  23
<212>  DNA
<213>  Artificial

<220>
<223>  Artificial

<400>  62
aaatcatctt aggagcacca tca                                              23


<210>  63
<211>  20
<212>  DNA
<213>  Artificial

<220>
<223>  Artificial

<400>  63
aaaaggagaa gcgaggaagg                                                  20


<210>  64
<211>  19
<212>  DNA
<213>  Artificial

<220>
<223>  Artificial

<400>  64

cttcctcttc gcttggtga                                                    19


<210>  65
<211>  25
<212>  DNA
<213>  Artificial

<220>
<223>  Artificial

<400>  65
atggaggaaa gtgatagtaa gagaa                                            25


<210>  66
<211>  22
<212>  DNA
<213>  Artificial

<220>
<223>  Artificial

<400>  66
gaggagagtg tgctaagtgg tg                                               22


<210>  67
<211>  19
<212>  DNA
<213>  Artificial

<220>
<223>  Artificial

<400>  67
aagatttttg tggtgggaa                                                   19


<210>  68
<211>  19
<212>  DNA
<213>  Artificial

<220>
<223>  Artificial

<400>  68
actcaatagg tcgcaatgg                                                   19


<210>  69
<211>  19
<212>  DNA
<213>  Artificial

<220>
<223>  Artificial

<400>  69
cacgtttctc tacggggac                                                   19


<210>  70
<211>  20

<212>  DNA
<213>  Artificial

<220>
<223>  Artificial

<400>  70
ctgcctttct gttgaactcc                                                    20


<210>  71
<211>  21
<212>  DNA
<213>  Artificial

<220>
<223>  Artificial

<400>  71
gcgtgtgact gatggaagaa g                                                  21


<210>  72
<211>  20
<212>  DNA
<213>  Artificial

<220>
<223>  Artificial

<400>  72
tcccaccgag tggagaatac                                                    20

## Description

### CROSS REFERENCE TO RELATED APPLICATION

This application claims the benefits of the Taiwan Patent Application Serial Number 100130368, filed on August 24, 2011, the subject matter of which is incorporated herein by reference.

### BACKGROUND OF THE INVENTION

### 1. Field of the Invention

The present invention relates to a method and a kit for identifying Phalaenopsis varieties. More specifically, the present invention relates to a method and a kit for identifying Phalaenopsis varieties by using microsatellite primer pairs.

### 2. Description of Related Art

Phalaenopsis is one of the top export floricultural goods from Taiwan. The export value of Phalaenopsis was about 99 million dollars in 2011, occupying 58% of the total export value for export flowers in Taiwan. At present, Taiwan has the vast Phalaenopsis orchid hybrids in the world, and has world-leading cultivation skills and breeding technique for modem, novel varieties bring about the great success. Therefore, Phalaenopsis is one of the most economically important floral crops in Taiwan; its economic value is most observable in areas such as commercial flask seedling, Phalaenopsis plantlet, pot flower, and cut flower.

The considerable number of Phalaenopsis varieties makes it necessary to identify the Phalaenopsis varieties for existing varieties, rare varieties, even the new varieties so as to distinguish the Phalaenopsis varieties and its economic value. In tradition, the main method used for identifying the Phalaenopsis varieties is based on the appearance , including flower color, pattern, flower size, flower count, numbers of spike, branching, stem height, etc. Nevertheless, it usually faces the problems about the serious mutations on flower shape and color of Phalaenopsis; or the pattern features affected by environment factors and the developmental stage of plants easily while using the traditional identification method. Meanwhile, not only do the mutations exist in the same variety, but also the mimic appearance exists in different varieties. Hence, resulting in the errors showed on the identification results. Besides, the traditional method is hard to apply on distinguishing the Phalaenopsis plantlet, and the time needed from plantlet to flowering is longer in Phalaenopsis. As a result, 1-3 years of waiting time is required before Phalaenopsis identification can begin.

Therefore, there is an urgent need to develop a method and a kit for identifying Phalaenopsis varieties, in hope that Phalaenopsis variety can be identified quicker at the early developmental stage; and infringement or tortious use of the variety can be reduced in order to protect the breeder's rights.

### SUMMARY OF THE INVENTION

The object of the present invention is to provide a kit for identifying Phalaenopsis varieties so as to identify the Phalaenopsis varieties.

Another object of the present invention is to provide a method for identifying Phalaenopsis varieties so that Phalaenopsis variety can be identified quicker at the early developmental stage.

To achieve the aforementioned objects, the present invention provides a kit for identifying Phalaenopsis varieties, comprising: at least one microsatellite primer pair, wherein each of the microsatellite primer pair comprises: nucleotides with 75-100% identity to the sequences represented by a primer pair selected from the following groups: a first primer pair comprises the sequences represented by SEQ ID NO: 1 and SEQ ID NO: 2; a second primer pair comprises the sequences represented by SEQ ID NO: 3 and SEQ ID NO: 4; a third primer pair comprises the sequences represented by SEQ ID NO: 5 and SEQ ID NO: 6; a fourth primer pair comprises the sequences represented by SEQ ID NO: 7 and SEQ ID NO: 8; a fifth primer pair comprises the sequences represented by SEQ ID NO: 9 and SEQ ID NO: 10; a sixth primer pair comprises the sequences represented by SEQ ID NO: 11 and SEQ ID NO: 12; a seventh primer pair comprises the sequences represented by SEQ ID NO: 13 and SEQ ID NO: 14; an eighth primer pair comprises the sequences represented by SEQ ID NO: 15 and SEQ ID NO: 16; a ninth primer pair comprises the sequences represented by SEQ ID NO: 17 and SEQ ID NO: 18; a tenth primer pair comprises the sequences represented by SEQ ID NO: 19 and SEQ ID NO: 20; an eleventh primer pair comprises the sequences represented by SEQ ID NO: 21 and SEQ ID NO: 22; a twelfth primer pair comprises the sequences represented by SEQ ID NO: 23 and SEQ ID NO: 24; a thirteenth primer pair comprises the sequences represented by SEQ ID NO: 25 and SEQ ID NO: 26; a fourteenth primer pair comprises the sequences represented by SEQ ID NO: 27 and SEQ ID NO: 28; a fifteenth primer pair comprises the sequences represented by SEQ ID NO: 29 and SEQ ID NO: 30; a sixteenth primer pair comprises the sequences represented by SEQ ID NO: 31 and SEQ ID NO: 32; a seventeenth primer pair comprises the sequences represented by SEQ ID NO: 33 and SEQ ID NO: 34; an eighteenth primer pair comprises the sequences represented by SEQ ID NO: 35 and SEQ ID NO: 36; a nineteenth primer pair comprises the sequences represented by SEQ ID NO: 37 and SEQ ID NO: 38; a twentieth primer pair comprises the sequences represented by SEQ ID NO: 39 and SEQ ID NO: 40; a twenty-first primer pair comprises the sequences represented by SEQ ID NO: 41 and SEQ ID NO: 42; a twenty-second primer pair comprises the sequences represented by SEQ ID NO: 43 and SEQ ID NO: 44; a twenty-third primer pair comprises the sequences represented by SEQ ID NO: 45 and SEQ ID NO: 46; a twenty-fourth primer pair comprises the sequences represented by SEQ ID NO: 47 and SEQ ID NO: 48; a twenty-fifth primer pair comprises the sequences represented by SEQ ID NO: 49 and SEQ ID NO: 50; a twenty-sixth primer pair comprises the sequences represented by SEQ ID NO: 51 and SEQ ID NO: 52; a twenty-seventh primer pair comprises the sequences represented by SEQ ID NO: 53 and SEQ ID NO: 54; a twenty-eighth primer pair comprises the sequences represented by SEQ ID NO: 55 and SEQ ID NO: 56; a twenty-ninth primer pair comprises the sequences represented by SEQ ID NO: 57 and SEQ ID NO: 58; a thirtieth primer pair comprises the sequences represented by SEQ ID NO: 59 and SEQ ID NO: 60; a thirty-first primer pair comprises the sequences represented by SEQ ID NO: 61 and SEQ ID NO: 62; a thirty-second primer pair comprises the sequences represented by SEQ ID NO: 63 and SEQ ID NO: 64; a thirty-third primer pair comprises the sequences represented by SEQ ID NO: 65 and SEQ ID NO: 66; a thirty-fourth primer pair comprises the sequences represented by SEQ ID NO: 67 and SEQ ID NO: 68; a thirty-fifth pair comprises the sequences represented by SEQ ID NO: 69 and SEQ ID NO: 70; and a thirty-sixth primer pair comprises the sequences represented by SEQ ID NO: 71 and SEQ ID NO: 72.

Thus, particularly a kit of the invention for identifying a Phalaenopsis variety comprises at least one microsatellite primer pair, wherein said primer pair is selected from a primer pair of SEQ ID NO: 1 and SEQ ID NO: 2; a primer pair of SEQ ID NO: 3 and SEQ ID NO: 4; a primer pair of SEQ ID NO: 5 and SEQ ID NO: 6; a primer pair of SEQ ID NO: 7 and SEQ ID NO: 8; a primer pair of SEQ ID NO: 9 and SEQ ID NO: 10; a primer pair of SEQ ID NO: 11 and SEQ ID NO: 12; a primer pair of SEQ ID NO: 13 and SEQ ID NO: 14; a primer pair of SEQ ID NO: 15 and SEQ ID NO: 16; a primer pair of SEQ ID NO: 17 and SEQ ID NO: 18; a primer pair of SEQ ID NO: 19 and SEQ ID NO: 20; a primer pair of SEQ ID NO: 21 and SEQ ID NO: 22; a primer pair of SEQ ID NO: 23 and SEQ ID NO: 24; a primer pair of SEQ ID NO: 25 and SEQ ID NO: 26; a primer pair of SEQ ID NO: 27 and SEQ ID NO: 28; a primer pair of SEQ ID NO: 29 and SEQ ID NO: 30; a primer pair of SEQ ID NO: 31 and SEQ ID NO: 32; a primer pair of SEQ ID NO: 33 and SEQ ID NO: 34; a primer pair of SEQ ID NO: 35 and SEQ ID NO: 36; a primer pair of SEQ ID NO: 37 and SEQ ID NO: 38; a primer pair of SEQ ID NO: 39 and SEQ ID NO: 40; a primer pair of SEQ ID NO: 41 and SEQ ID NO: 42; a primer pair of SEQ ID NO: 43 and SEQ ID NO: 44; a primer pair of SEQ ID NO: 45 and SEQ ID NO: 46; a primer pair of SEQ ID NO: 47 and SEQ ID NO: 48; a primer pair of SEQ ID NO: 49 and SEQ ID NO: 50; a primer pair of SEQ ID NO: 51 and SEQ ID NO: 52; a primer pair of SEQ ID NO: 53 and SEQ ID NO: 54; a primer pair of SEQ ID NO: 55 and SEQ ID NO: 56; a primer pair of SEQ ID NO: 57 and SEQ ID NO: 58; a primer pair of SEQ ID NO: 59 and SEQ ID NO: 60; a primer pair of SEQ ID NO: 61 and SEQ ID NO: 62; a primer pair of SEQ ID NO: 63 and SEQ ID NO: 64; a primer pair of SEQ ID NO: 65 and SEQ ID NO: 66; a primer pair of SEQ ID NO: 67 and SEQ ID NO: 68; a primer pair of SEQ ID NO: 69 and SEQ ID NO: 70; and a primer pair of SEQ ID NO: 71 and SEQ ID NO: 72, or a primer pair having at least 75% identity thereto.

A primer pair present in the kit may therefore have at least 80, 85, 90, 95, 96, 97, 98 or 99% sequence identity with any one of the primer pairs set out above.

Identity may be determined using the Bestfit program of the Genetics Computer Group Version 10 software package from the University of Wisconsin. The Pogram uses the local hand algorithm of Smith and Waterman with the default values: Gap creation penalty=8, Gap extension penalty=2, Average match=2.912, Average mismatch=2.003.

Thus, as described above, a primer pair typically comprises two primers and as discussed above a primer pair in the kit of the invention may consist of two primers having the specific sequences as set out in the pairs of SEQ ID NOs listed above, or may have at least 75% identity to any of those sequences. Thus, in any primer pair, either or both primers may have at least 75 % identity to any of the primers in any of the specific primer pair sequences. Hence, for example for a primer pair of SEQ ID NO. 1 and SEQ ID NO. 2, the kit may comprise primers with these specific sequences or may comprise one primer with at least 75% identity to SEQ ID NO. 1 and the other primer having the sequence of SEQ ID NO. 2, or may comprise one primer having the sequence of SEQ ID NO.1 and the other primer with at least 75% identity to SEQ ID NO. 2 or may comprise one primer with at least 75% identity to SEQ ID NO. 1 and the other primer with at least 75% identity to SEQ ID NO. 2.

The kit of the invention may comprise at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 primer pairs.

Particularly, the kit may comprise primer pairs of SEQ ID NO. 1 and SEQ ID NO. 2, SEQ ID NO. 3 and SEQ ID NO. 4, SEQ ID NO. 5 and SEQ ID NO. 6, SEQ ID NO. 7 and SEQ ID NO. 8, SEQ ID NO. 13 and SEQ ID NO. 14, SEQ ID NO. 31 and SEQ ID NO. 32 and SEQ ID NO. 37 and SEQ ID NO. 38 or primer pairs having at least 75% identity thereto.

Typically, one of the primers in the primer pair is a forward primer and the other primer is a reverse primer.

Furthermore, the present invention provides a method by using the aforementioned kit for identifying Phalaenopsis varieties, comprising the following steps: (A) providing a tissue sample of the to be tested Phalaenopsis variety, and isolating the genomic DNA from the tissue sample; (B) obtaining the genotype of a tested Phalaenopsis variety by using at least one microsatellite primer pair to amplify the genomic DNA of the tissue sample and (C) comparing the genotype obtained from the tested Phalaenopsis variety with a known database of the Phalaenopsis genotype to identify the variety of the tested sample.

Specifically, the invention provides a method for identifying a Phalaenopsis variety comprising
A) providing a tissue sample of a Phalaenopsis variety and isolating genomic DNA therefrom,
B) determining the genotype of said Phalaenopsis variety using at least one microsatellite primer pair as set out above to amplify the genomic DNA and
C) comparing said genotype with a known database of Phalaenopsis variety genotypes to identify the variety of the tested Phalaenopsis.

In the kit and the method for identifying Phalaenopsis varieties of the present invention, it only requires a very small amount of genomic DNA isolated from plant tissue to identify the Phalaenopsis varieties quickly by using microsatellite primer pair. Compared with the DNA molecular marker of the techniques such as amplified fragment length polymorphism (AFLP), random amplified polymorphic DNA (RAPD), and restriction fragment length polymorphism (RFLP), the microsatellite sequence of the present invention has the advantages of being handled relatively quickly and simplistically (only 3 to 7 days is needed for the identification), high polymorphism, high reproducibility, and applying to the automatic analysis with high throughput. Besides, the kit and the method for identifying Phalaenopsis varieties of the present invention are not affected by the growth environment of plants. Therefore, the kit and the method can be applied for detecting the non-flowered plants, plantlet, or flask seedling, and for identifying the Phalaenopsis varieties at the early developmental stage. Furthermore, the kit and the method for identifying Phalaenopsis varieties provided by the present invention can give a unique DNA molecular code for every Phalaenopsis variety, increasing the speed and the accuracy for identifying Phalaenopsis varieties.

The tissue sample used in the method may be root, stem, flower or leaves

In the kit and the method for identifying Phalaenopsis varieties of the present invention, the better, used microsatellite primer pairs is a set of the primer pairs which comprises any one nucleotide sequence selected from the group consisting of the first primer pair to the thirty-sixth primer pair. In addition, the 5' end or the 3' end of the sequence of these primer pairs can further add several nucleotides (1 to 15 different). For example, at least 1, 2, 3, 4, 5, 6, 7, 8, 9 or 10 nucleotides may be added to either or both of the 5' and/or 3' ends of a primer in a primer pair. Preferably, the best, used primer pairs are a set of the primer pairs which is selected from the group consisting of the first primer pair to the thirty-sixth primer pair.

Moreover, in the kit and the method for identifying Phalaenopsis varieties of the present invention, at least one microsatellite primer pair includes a forward microsatellite primer and a reverse microsatellite primer. Preferably, one of the 5' ends of the forward microsatellite primer or the reverse microsatellite primer labeled with a fluorescent reagent. After the primer pair labeling with the fluorescent reagent and then obtaining a amplification product by polymerase chain reaction (PCR), the genotype of Phalaenopsis varieties can be determined by DNA analyzer. A preferred arrangement is that different microsatellite primer pairs labeled with different fluorescent reagents and the amplification products with different fluorescent reagents labeled are mixed into the same tube before conducting the genotyping analysis. The fluorescent reagent labeled at either the forward primer or the reverse primer can be a common fluorescent labeling reagent used in this technical field, such as HEX, FAM, and NED.

Also, in the method for identifying Phalaenopsis varieties of the present invention, step (B) involves using a DNA analysis technique commonly known in this field of technology to analyze the genomic DNA of the tissue sample; a preferred embodiment of this aspect of the invention is to use polymerase chain reaction (PCR) to analyze the genomic DNA of a tissue sample, using at least one microsatellite primer pair. In addition, the size of the amplified fragment can be used to differentiate Phalaenopsis varieties.

### BRIEF DESCRIPTION OF THE DRAWINGS

FIG. 1 is the preferred embodiment of the present invention showing the results of *Dtps.* Taida Firebird 'Taida Red Rose' and *Dtps.* Sogo Meili 'SOGO F1751' by DNA analyzer; and
FIG. 2 is the preferred embodiment of the present invention showing the result of similarity cluster analysis for identifying Phalaenopsis varieties by using microsatellite primer pairs.

### DETAILED DESCRIPTION OF THE PREFERRED EMBODIMENT

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

### <Isolating the genomic DNA from the tissue sample of Phalaenopsis variety>

Root, stem, leaf, or flower tissues of Phalaenopsis variety were collected for analyzing the genotypes of Phalaenopsis varieties. The tissues were ground into powder by a tissue grater or pulverized in liquid nitrogen, the genomic DNA was mini-extracted by use of the CTAB (cetyltrimethylammonium bromide) method or the BioKit plant genomic DNA purification kit and diluted to a concentration of 3 ng/µL and stored at -20°C waiting for use.

51 Phalaenopsis varieties (as shown in the following Table 2) are collected for the present embodiment, for which applications for the plant breeder's rights are currently pending or already obtained in Taiwan. About 0.2 g leaf tissue of Phalaenopsis varieties are provided here for demonstration, which were ground into fine powder with the presence of liquid nitrogen. The plant DNAs were isolated using the BioKit Plant Genomic DNA Purification Kit (BioKit Biotechnology Incorporation) or the QIAGEN DNeasy Plant Mini Kit (QIAGENE), and were diluted to 3 ng/µL in concentration and stored at -20°C before use.

### <Analyzing the genomic DNA via PCR>

First, the 5 µL genomic DNA (3 ng/µL) of the Phalaenopsis tissue sample was taken, and the 2.5 µL, 10X PCR buffer (VIOGENE); 2.5 µL, 1% Tween 20; 1.0 µL, 2.5 mM dNTP; 1.0 µL, 5 µmol forward primer of microsatellite primer (as shown in the following Table 1) labeled with a fluorescent reagent; 1.0 µL, 5 µmol reverse primer of microsatellite primer (as shown in the following Table 1 without labeling a fluorescent reagent); 0.2 µL, 5 U DNA polymerase (PureTaq DNA polymerase, VIOGENE) were added. The sterile water was added to count for 25 µL for the total volume and proceeded through the PCR amplification. Wherein the conditions of PCR amplification were as follows: first, reacted 5 minutes at 94°C; 40 cycles were repeated and each cycle was designed to react 30 seconds at 94°C; reacted 30 seconds at annealing temperature (as shown in the following Table 1); reacted 40 seconds at 72°C; finally, reacted 5 minutes at 72°C and the reaction stopping temperature was 4°C.

**Table 1**

| **Primer pair No.** | **SEQ ID NO:** | **Forward and reverse microsatellite primer sequence** | **Tm (°C)** | **Expected size (bp)** | **Fluorescent reagent** |
|---|---|---|---|---|---|
| 1 | 1 | F: GCCCAATTTTCAGTTTTCCTTC | 55 | 400 | FAM/NED |
| | 2 | R: TTGCTTCATTGTTTTTCCTTTCAC | | | |
| 2 | 3 | F: TCTTTGTGTGTGTGTGCGTG | 55 | 179 | FAM |
| | 4 | R: GTGTCCCGTAGACTTCCCG | | | |
| 3 | 5 | F: GGCGAGCGATTGGTGTGC | 55 | 283 | FAM |
| | 6 | R: TTTTTTCCTCATTCGGTCAGGC | | | |
| 4 | 7 | F: CTTGAGCGGATTGGGTCTTA | 51 | 268 | HEX |
| | 8 | R: CACTCAGCCAACGCATCGAA | | | |
| 5 | 9 | F: GCAATTTGACTACCCCCTCTC | 51 | 167 | TET |
| | 10 | R: GCATGGATTAACCGTTTTTCTT | | | |
| 6 | 11 | F: TTCGGACTACAAGGCGTACC | 55 | 236 | FAM |
| | 12 | R: CATTTGGCTCAGGCTCAGTAC | | | |
| 7 | 13 | F: CTCATTCGGTCAGGCATTTC | 55 | 172 | HEX |
| | 14 | R: AGCTGCTTCCCAAGTGATTC | | | |
| 8 | 15 | F: GAATGAGGGCACCAAATCT | 49 | 338 | HEX/NED |
| | 16 | R: TCCATTAACCCCGAGAAAAA | | | |
| 9 | 17 | F: GGAACCAACAAGAAGAAGAAGC | 51 | 232 | FAM |
| | 18 | R: GCATCGCAGAGAACAGAGAGC | | | |
| 10 | 19 | F: GGGATGGAGGGATTTGAGAT | 50 | 287 | FAM |
| | 20 | R:AAGCACAGGAAACGCAAACT | | | |
| 11 | 21 | F: GAGCCCGATCACAACGACC | 55 | 325 | FAM |
| | 22 | R: GTGACTCCGTTCCATGCCTC | | | |
| 12 | 23 | F: AAGATGGGTAGTATGTGAGAATGC | 51 | 232 | HEX |
| | 24 | R: ATCAGCCACAAAGCAGAAGC | | | |
| 13 | 25 | F: GGAAGCAGGCAAAGGTATGA | 51 | 218 | FAM |
| | 26 | R: TTGCTGTCTCCCTCATCTGC | | | |
| 14 | 27 | F: AGCCTACCTGATTCGCCATA | 51 | 211 | HEX |
| | 28 | R: CCCATTCATCCCCTCTCTCT | | | |
| 15 | 29 | F: ACACTCCCTGCCAATGGTAA | 51 | 250 | NED |
| | 30 | R: TCCATTTCCTTCATTTTGTGC | | | |
| 16 | 31 | F: GGTCAGCCTCCTTCATCAGA | 50 | 229 | HEX |
| | 32 | R: TGGTCATGTGGTGTGTTTTTA | | | |
| 17 | 33 | F: TAGTGGTGGTGGCGATGATA | 50 | 166 | FAM |
| | 34 | R: CTAAATAATGCCAAGGGGTAACT | | | |
| 18 | 35 | F: GTAATCACAACTAAACAGGGCTAA | 49 | 240 | HEX |
| | 36 | R: ATGATGATGAGGAGGAGTAGATTC | | | |
| 19 | 37 | F: TGGTCTCTGTCGTCACTTGG | 50 | 219 | FAM |
| | 38 | R: AAGAATTACACCGCCGATCA | | | |
| 20 | 39 | F: CATTAGTGGTCTTTCCCTGTG | 47 | 300 | FAM |
| | 40 | R: AAGAGTGGATGTTTTAGTTTCG | | | |
| 21 | 41 | F: GCTTTCTGCTTATCGTTCCA | 49 | 247 | FAM |
| | 42 | R: GCTTACAGTGGACGGGCT | | | |
| 22 | 43 | F: TGGAGGGTTAGAAGCAAGGA | 50 | 223 | FAM |
| | 44 | R:TCATCATCCCTCTTCCTCGT | | | |
| 23 | 45 | F: GTTTATCTTCATGTGGTCCTCG | 51 | 256 | HEX |
| | 46 | R: CTTACTTGTCCACCCCCATT | | | |
| 24 | 47 | F: GGCTCACCTTCCTTCTCCTT | 51 | 260 | NED |
| | 48 | R:GCTAAACCCTAAACCCTTGTGT | | | |
| 25 | 49 | F: CAACTCCCAAAGCCTCAACT | 50 | 185 | HEX |
| | 50 | R: GACCCACACCATTCCATCAC | | | |
| 26 | 51 | F: CTAACTCTCTTTCCTTGCTCCTT | 50 | 195 | FAM |
| | 52 | R: CGTTTCTTCCTCTCTCACACTC | | | |
| 27 | 53 | F: TTGGATGGTAGGTATGAGTATGC | 50 | 249 | FAM |
| | 54 | R: TGATTGTTCTTCCCGTCTGTT | | | |
| 28 | 55 | F: CCTCGTGCTTTCCTGTTCAT | 50 | 215 | FAM |
| | 56 | R: AGTTTTGGGTGCTGCTATGC | | | |
| 29 | 57 | F: ATGAATCGTTTGGTATGTATGTGA | 50 | 220 | NED |
| | 58 | R: ATTGTTCATTGCTGCTGCTG | | | |
| 30 | 59 | F: AAGTTATCCAAATGACCCCAG | 50 | 272 | FAM |
| | 60 | R: AAACTTCGGTGCCTGAGAGA | | | |
| 31 | 61 | F: CCGCTCTTCCAGTTTCGTT | 52 | 187 | NED |
| | 62 | R: AAATCATCTTAGGAGCACCATCA | | | |
| 32 | 63 | F: AAAAGGAGAAGCGAGGAAGG | 50 | 163 | HEX |
| | 64 | R: CTTCCTCTTCGCTTGGTGA | | | |
| 33 | 65 | F: ATGGAGGAAAGTGATAGTAAGAGAA | 50 | 151 | FAM |
| | 66 | R: GAGGAGAGTGTGCTAAGTGGTG | | | |
| 34 | 67 | F: AAGATTTTTGTGGTGGGAA | 50 | 171 | NED |
| | 68 | R: ACTCAATAGGTCGCAATGG | | | |
| 35 | 69 | F: CACGTTTCTCTACGGGGAC | 50 | 262 | HEX |
| | 70 | R: CTGCCTTTCTGTTGAACTCC | | | |
| 36 | 71 | F: GCGTGTGACTGATGGAAGAAG | 50 | 357 | FAM |
| | 72 | R: TCCCACCGAGTGGAGAATAC | | | |

Electrophoresis analysis was performed on a 2 to 3 % agarose gel, confirming whether the products amplified via PCR by the microsatellite primer pairs existed or not. The amplified PCR products with different fluorescent reagents labeled were mixed, and sent to any Biotechnology Company to identify the genotypes of Phalaenopsis varieties derived from microsatellite molecular markers by using DNA analyzer. Finally, the allele sizes were scored using Peak Scanner or GeneMapper software to analyze the genotypes of Phalaenopsis varieties.

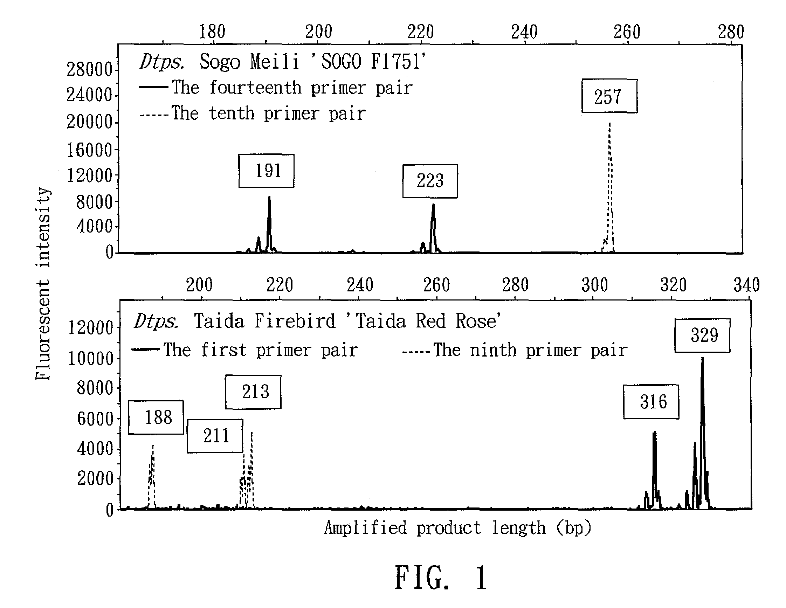

In the present embodiment, the 19 sets of the microsatellite primer pairs (as shown in the following Table 3) were used to perform PCR amplification and analysis with regard to the 51 Phalaenopsis varieties (as shown in the following Table 2). Product electrophoresis was carried out on a 3 % agarose gel, for the purpose of checking whether the products amplified via PCR existed in the range of expected size (as shown in the aforementioned Table 1). The 2 to 3 amplified PCR products labeled with different fluorescent reagents were mixed in the same tube, and sent to any Biotechnology Company to identify the genotypes of the 51 Phalaenopsis varieties derived from the 19 sets of the microsatellite molecular markers by DNA analyzer (ABI PRISM 3730 DNA analyzer). The results for analyzing *Dtps.* Taida Firebird 'Taida Red Rose' by using the first primer pair and the ninth primer pair, and for analyzing *Dtps.* Sogo Meili 'SOGO F1751' by using the tenth primer pair and the fourteenth primer pair via DNA analyzer were shown in Figure 1.

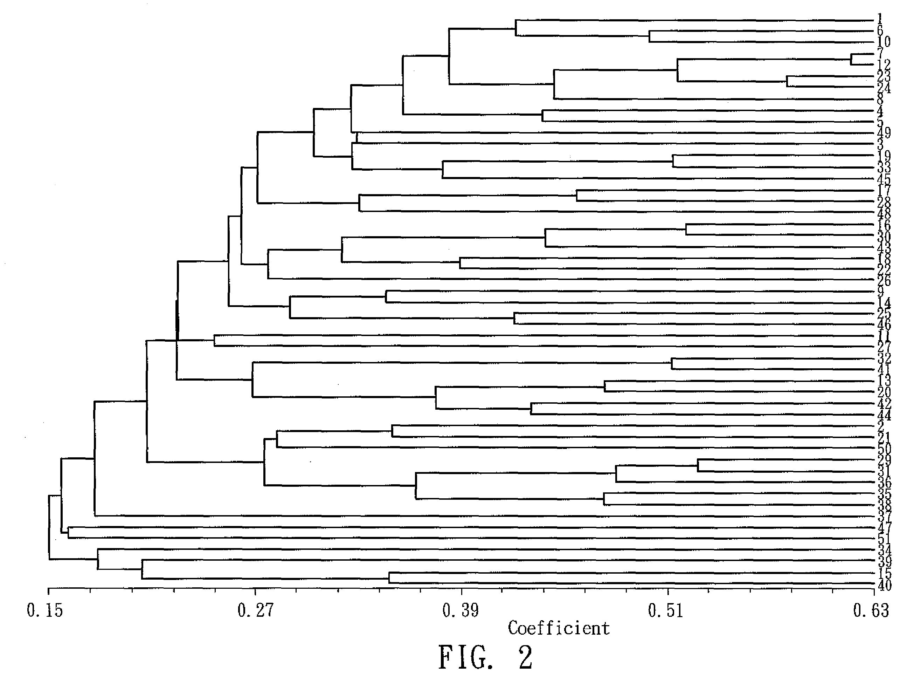

The identified results were recored via Peak Scanner software and the genotypes obtained via analysis by the 19 sets of the microsatellite primer pairs for every Phalaenopsis variety afterward, as shown in the following Table 2 (only exhibited the analytical results by the first, the second, and the third primer pairs). According to the allele observed in the Phalaenopsis variety: the specific alleles were represented as "1" (present) and "0" (absent). The similarity analysis was performed to analyze the varieties using NYSYS software and the results are shown in Figure 2. The 1 to 51 numbers shown in Figure 2 represent the 51 Phalaenopsis varieties. The Figure 2 are the results for identifying the genotypes using 19 sets of the microsatellite molecular markers, and it can discriminate the 51 Phalaenopsis varieties which were applied for registration in Taiwan or granted the plant variety rights in Taiwan. Therefore, the results demonstrated that the microsatellite molecular markers labeled with fluorescent reagents of the present invention can identify the Phalaenopsis varieties successfully.

**Table 2**

| | | **Amplified product size (bp)** | | |
|---|---|---|---|---|
| **No.** | **Phalaenopsis varieties** | **The first primer pair** | **The second primer pair** | **The third pair primer** |
| 1 | *P*. Yu Pin pearl 'YPM131' | 325, 329 | 166, 177, 200 | N^{a} |
| 2 | *P*. Taihort Gem 'TSC 13 8' | 320, 328 | 142, 165 | 291 |
| 3 | *P*. Sogo Imp 'SOGO F-940' | 320 | 200 | 281,287 |
| 4 | *P*. Join Grace 'TH.288-4' | 320, 325, 320, 325, | 164, 190 | 299 |
| 5 | *P*. Sogo Firework 'SOGO F-802' | 320, 343 | 164, 198 | 303, 308 |
| 6 | *P*. Sogo Gold 'SOGO F-1046' | 328337 | 165,200 | 296,299 |
| 7 | *P*. Tai Lin Prince 'Bei ji guang V265' | 320, 337 | N | 299, 305 |
| 8 | *P*. Tai Ling Queen 'Queen V6' | 319, 325, 337 | 177 | N |
| 9 | *P*. Dou-dii Pride 'Mei Dar Red Star' | 320, 328 | 164, 166 | 283,299 |
| 10 | *P*. Sogo Gold 'SOGO F1047' | 309, 328, 337 | 165, 177 | 299 |
| 11 | *P*. Classic Beauty 'Color Butterfly' | 318, 343 | 169, 177 | 283,298 |
| 12 | *P.* Sogo Yukidian 'Shiuh-Dong Whishkey' | 320, 337 | 177 | 299 |
| 13 | *P*. Lawrence of Arabia 'NCYU Sandy' | 318, 337 | 164, 177 287 | 281, 285, |
| 14 | *P*. Ho's Colourful Bubbles 'The Pride of Taiwan' | 318, 320, 328 | 182 | 283, 287, 305 |
| 15 | *P*. Tainung No. 1 'Pixie' | 309, 343 | 142 | 305, 307 |
| 16 | *Dtps.* Sogo Romantic 'SOGO F-982' | 309, 319, | 165 | 283, 295, |
| | | 327, 329 | | 308 |
| 17 | *Dtps.* Bread Rose 'Lih Jiang Firebird' | 328, 337 | 165 | 283, 291, 299 |
| 18 | *Dtps.* Sogo Yoshida 'SOGO F-1302' | 319, 337 | 176 | 283 |
| 19 | *Dtps.* Lepoard Prince 'SOGOF-1138' | 320, 328 | 165, 176 | 291 299 307 299, |
| 20 | *Dtps.* Sogo Pride 'SOGOF-1016' | 318, 337 | 176 | 285, 293, 299 |
| 21 | *Dtps.* Sogo vivien 'SOGO F-858' | 320, 328 | 142, 165 | 291, 303 |
| 22 | *Dtps.* Nobby's Pink Lady 'Pingtung Queen' | 320, 327 | 176, 198 | 282 |
| 23 | P. Sogo Muyudian 'LW9441 ' 'Melor CL331' | 309, 320, 337 | 166, 177 | 297, 299 |
| 24 | P. Unimax Glory 'LW9509' 'White Ribbon CL369' | 318, 320, 337 | 166, 177 | 296 |
| 25 | P. Unimax Moonlight 'Fortune CL577' | 337 | 184, 200 | 283, 287, 289, 299 |
| 26 | P. Unimax Sakurahime 'Princess Sakura CL805' | 327 | 177, 200 | N |
| 27 | *Dtps.* Unimax Cradle 'SWR9501' 27 'Lullaby CL904' | 310, 337 | 177, 180 | N |
| 28 | *Dtps.* Sogo Moonhalo 'Sogo F-1061' | 322, 337 | 165, 177, 200 | 283, 291, 283, 291, 299 |
| 29 | *Dtps.* Sogo Alice 'Sogo F-1199' | 320, 339 | 165, 177 | 285, 295, 303 295, |
| 30 | *Dtps.* Sogo Gumbo 'Sogo F-981' | 309, 320, 327 | 165, 177 | 283,295 |
| 31 | *Dtps.* Sogo Weddung 'Sogo F-879' | 366 | 142, 165, 177 | 285,295 |
| 32 | *Dtps.* Sogo Gotris 'Sogo F-1307' | 325, 339 | 142 | 298 |
| 33 | *Dtps.* Leopard Prince 'SOGO F-977' | 320, 329 | 161, 200 | 283, 295, 307 295, |
| 34 | *P*. Sogo Golden Timothy 'Sogo F-1034' | 314, 337, 366 | 168 | 281, 305 |
| 35 | *P*. Taisuco Eros 'Pink Butterfly' | 320, 329, 339 | 165, 176, 185, 200 | 283, 291, 303 |
| 36 | *P*. Taisuco Pioneer 'Vanguard' | 317, 327, 336 | 165, 177 | 285, 303 |
| 37 | *P*. Taisuco Snowing 'TSC 139' | 309 | 177 | 293, 303 |
| 38 | *P*. Taisuco Stellar 'Red Pearl' | 328, 339 | 142,200 | 291, 303 |
| 39 | *P*. Sogo Fairyland 'Sogo F-842' | 320, 337, 343 | 141, 176 | 297, 305 |
| 40 | *P*. SOGO Venis 'SOGO F1314' | 337 | 180 | 289, 305 |
| 41 | *Dtps.* Sogo Gotris 'SOGO F1248' | 319 325 339 325, | 141, 176 | 297 |
| 42 | *Dtps*. Sogo Meili 'SOGO F1751' | 319, 328 | 176 | 293,295 |
| 43 | *Dtps.* Sogo Melody 'SOGO F1951' | 318, 322, 329, 337 | 165, 186 | 283,295 |
| 44 | *P*. Sogo Lawrence 'SOGO F-1982' | 318, 325 | 176, 200 | 283, 291, 299 |
| 45 | *Dtps.* Sogo Passat 'Sogo F-1383' | 328, 336 | 176, 200 | 283, 291, 304 291, |
| 46 | *P*. Taida Smile 'Taida Little Green' | 316, 337 | 200 | 283, 295, 305 |
| 47 | *P*. Taida Lovely 'Taida Pink Swan' | 318, 327 | 165, 176 | 296, 299 |
| 48 | *Dtps.* Taida Firebird 'Taida Red Rose' | 316, 329 165, | 176 | 291, 303 |
| 49 | *Dtps.* Hsinying Mount 'Taida Snow Peach' | 320, 337 | 166, 176, 185,200 | 296, 299, 312 |
| 50 | *Dtps.* SOGO Breeze 'SOGO F1621' | 320, 339, 365' 339, | 142, 176 | 309 |
| 51 | *P*. Sogo Relex 'SOGO F1661' | 323, 337 | 165 | 287, 312 |

| | | | | |
|---|---|---|---|---|
| ^{a}N: represents the Phalaenopsis variety did not have PCR amplified product by the microsatellite primer pair. | | | | |

In addition, the frequency and the number of the alleles existed in the 51 Phalaenopsis varieties by each microsatellite primer pair were analyzed, and the PIC value that represents the discrimination power of the microsatellite molecular marker was calculated according to the genotyping results. The PIC values of the 19 sets of the microsatellite molecular markers are ranging from 0.62 (the sixth primer pair) to 0.95 (the twenty-third primer pair). In the statistics of unique genotype number, 49 different unique genotypes were obtained in the 51 varieties by using the seventh primer pair. It represents that one set of the microsatellite primer pair- the seventh primer pair can identify 49 varieties (as shown in the following Table 3). Hence, the results demonstrated that the microsatellite molecular markers of the present invention have high identification efficacy to distinguish the Phalaenopsis varieties.

**Table 3**

| Microsatellite primer pair | Fluorescent reagent | Amplified product length (bp) | Different genotype number^{a} | Allele number^{a} | PIC value^{b} |
|---|---|---|---|---|---|
| The first | FAM/NED | 309-366 | 43 | 20 | 0.9 |
| primer pair | | | | | |
| The second primer pair | HEX | 141-200 | 34 | 18 | 0.88 |
| The third primer pair | FAM | 281-312 | 40 | 20 | 0.92 |
| The fourth primer pair | HEX | 241-386 | 31 | 24 | 0.92 |
| The fifth primer pair | TET | 150-269 | 21 | 18 | 0.87 |
| The sixth primer pair | FAM | 208-236 | 6 | 5 | 0.62 |
| The seventh primer pair | HEX | 135-171 | 49 | 31 | 0.94 |
| The eighth primer pair | HEX/NED | 309-345 | 43 | 21 | 0.92 |
| The ninth primer pair | FAM | 183-233 | 42 | 27 | 0.88 |
| The tenth primer pair | FAM | 251-280 | 18 | 11 | 0.74 |
| The eleventh primer pair | FAM | 291-346 | 14 | 12 | 0.73 |
| The twelfth primer pair | HEX | 214-256 | 44 | 23 | 0.91 |
| The thirteenth primer pair | FAM | 192-242 | 39 | 19 | 0.86 |
| The fourteenth primer pair | HEX | 185-260 | 48 | 33 | 0.94 |
| The fifteenth primer pair | NED | 212-315 | 32 | 26 | 0.93 |
| The sixteenth primer pair | HEX | 226-280 | 45 | 27 | 0.92 |
| The nineteenth primer pair | FAM | 194-219 | 34 | 14 | 0.89 |
| The twenty-third primer pair | HEX | 198-255 | 47 | 32 | 0.95 |
| The twenty-fourth primer pair | NED | 219-330 | 35 | 24 | 0.9 |

| | | | | | |
|---|---|---|---|---|---|
| ^{a}Different genotype number and ^{a}Allele number: represent the genotype number and allele number obtained in the 51 Phalaenopsis varieties by each microsatellite primer pair. ^{b}PIC value: Polymorphism Information Content, represents the discrimination power of microsatellite molecular marker; the PIC value is larger, and the microsatellite molecular marker is more discriminative. | | | | | |

According to the results of the abovementioned Table 1 to Table 3 and Figure 1 to Figure 2, the microsatellite primer pair of the present invention has excellent identifying ability; the abovementioned Phalaenopsis varieties can be identified by using only a few sets of the microsatellite primer pairs. Moreover, identifying varieties can be performed without Phalaenopsis flowering. Therefore, the present invention can be used in the non-flowering plantlet for identifying at the early developmental stage as well as increasing the accuracy for identifying Phalaenopsis varieties.

On the other hand, the present invention providing the kit and the method for identifying Phalaenopsis varieties can give a unique DNA molecular code for every Phalaenopsis variety, construct a DNA database and reduce the possibility of the varieties suffered from infringement and tortious actions.

Here, some Phalaenopsis varieties were analyzed by using the microsatellite primer pairs (the first primer pair, the second primer pair, the third primer pair, the fourth primer pair, the seventh primer pair, the sixteenth primer pair, and the nineteenth primer pair) of the present invention to obtain the DNA molecular code of the genotype of Phalaenopsis varieties. The results are shown in the following Table 4.

**Table 4**

| **Varieties** | **The first primer pair** | | **The second primer pair** | | **The third primer pair** | | **The fourth primer pair** | | **The seventh primer pair** | | **The sixteenth primer pair** | | **The nineteenth primer pair** | | **Code** |
|---|---|---|---|---|---|---|---|---|---|---|---|---|---|---|---|
| *P*. Join Grace 'TH. 288-4' | 320, 325, 343 | Q | 164, 190 | G | 299 | F | 276, 282 | J | 153, 155 | P | 269, 271 | Q | 200, 213 | G | QGF JPQG |
| *P*. Tai Lin Prince 'Bei ji guang V265' | 320, 337 | G | N | M | 299, 305 | L | 241 | A | 144, 155, 161 | G | 257, 265, 269, 277 | K | 200, 213 | G | GML AGKG |
| *P*. Tai Ling Queen 'Queen V6' | 319, 325, 337 | O | 177 | D | N | R | 241, 251 | B | 146, 155, 163 | K | 265, 271, 279 | O | 200, 214, 216 | H | ODR BKOH |
| *P*. Sogo Yukidian 'Shiuh-Dong Whishkey' | 320, 337 | G | 177 | D | 299 | F | 241 | A | 153, 155, 164 | Q | 269, 271, 280 | R | 200 | C | GDF AQRC |

For example, when analyzing by using the first primer pair as the microsatellite primer pair, the letter "G" can represent genotype (320, 337); when analysis is using the second primer pair as the microsatellite primer pair, the letter "D" and "G" can represent genotype (177) and (164, 190) respectively. In other words, the different letters are obtained based on different genotypes by the analysis of each primer pair, and every analyzing result of primer pair is corresponding to a letter separately. In the present embodiment, identification by using 7 sets of microsatellite primer pairs can obtain the DNA molecular code composed of 7 letters. For instance, the results for identifying P. Join Grace 'TH.288-4' and *P.* Tai Lin Prince 'Bei ji guang V265' can show the unique DNA molecular codes- QGFJPQG and GMLAGKG, respectively by using 7 sets of the microsatellite primer pairs.

The more sets of microsatellite primer pairs are used in analysis, the more letters can be obtained to compose a specific molecular code. Based on this unique DNA molecular code, a DNA database of Phalaenopsis varieties can be constructed to reduce infringement or tortious use of the variety in order to protect breeder's rights.

Although the present invention has been explained in relation to its preferred embodiment, it is to be understood that many other possible modifications and variations can be made without departing from the spirit and scope of the invention as hereinafter claimed.

## Claims

1. A kit for identifying Phalaenopsis varieties, comprising:
at least one microsatellite primer pair, wherein each of the microsatellite primer pair comprises: nucleotides with 75-100% identity to the sequences represented by a primer pair selected from the following groups:
a first primer pair comprising the sequences represented by SEQ ID NO: 1 and SEQ ID NO: 2;
a second primer pair comprises the sequences represented by SEQ ID NO: 3 and SEQ ID NO: 4;
a third primer pair comprises the sequences represented by SEQ ID NO: 5 and SEQ ID NO: 6;
a fourth primer pair comprises the sequences represented by SEQ ID NO: 7 and SEQ ID NO: 8;
a fifth primer pair comprises the sequences represented by SEQ ID NO: 9 and SEQ ID NO: 10;
a sixth primer pair comprises the sequences represented by SEQ ID NO: 11 and SEQ ID NO: 12;
a seventh primer pair comprises the sequences represented by SEQ ID NO: 13 and SEQ ID NO: 14;
a eighth primer pair comprises the sequences represented by SEQ ID NO: 15 and SEQ ID NO: 16;
a ninth primer pair comprises the sequences represented by SEQ ID NO: 17 and SEQ ID NO: 18;
a tenth primer pair comprises the sequences represented by SEQ ID NO: 19 and SEQ ID NO: 20;
an eleventh primer pair comprises the sequences represented by SEQ ID NO: 21 and SEQ ID NO: 22;
a twelfth primer pair comprises the sequences represented by SEQ ID NO: 23 and SEQ ID NO: 24;
a thirteenth primer pair comprises the sequences represented by SEQ ID NO: 25 and SEQ ID NO: 26;
a fourteenth primer pair comprises the sequences represented by SEQ ID NO: 27 and SEQ ID NO: 28;
a fifteenth primer pair comprises the sequences represented by SEQ ID NO: 29 and SEQ ID NO: 30;
a sixteenth primer pair comprises the sequences represented by SEQ ID NO: 31 and SEQ ID NO: 32;
a seventeenth primer pair comprises the sequences represented by SEQ ID NO: 33 and SEQ ID NO: 34;
an eighteenth primer pair comprises the sequences represented by SEQ ID NO: 35 and SEQ ID NO: 36;
a nineteenth primer pair comprises the sequences represented by SEQ ID NO: 37 and SEQ ID NO: 38;
a twentieth primer pair comprises the sequences represented by SEQ ID NO: 39 and SEQ ID NO: 40;
a twenty-first primer pair comprises the sequences represented by SEQ ID NO: 41 and SEQ ID NO: 42;
a twenty-second primer pair comprises the sequences represented by SEQ ID NO: 43 and SEQ ID NO: 44;
a twenty-third primer pair comprises the sequences represented by SEQ ID NO: 45 and SEQ ID NO: 46;
a twenty-fourth primer pair comprises the sequences represented by SEQ ID NO: 47 and SEQ ID NO: 48;
a twenty-fifth primer pair comprises the sequences represented by SEQ ID NO: 49 and SEQ ID NO: 50;
a twenty-sixth primer pair comprises the sequences represented by SEQ ID NO: 51 and SEQ ID NO: 52;
a twenty-seventh primer pair comprises the sequences represented by SEQ ID NO: 53 and SEQ ID NO: 54;
a twenty-eighth primer pair comprises the sequences represented by SEQ ID NO: 55 and SEQ ID NO: 56;
a twenty-ninth primer pair comprises the sequences represented by SEQ ID NO: 57 and SEQ ID NO: 58;
a thirtieth primer pair comprises the sequences represented by SEQ ID NO: 59 and SEQ ID NO: 60;
a thirty-first primer pair comprises the sequences represented by SEQ ID NO: 61 and SEQ ID NO: 62;
a thirty-second primer pair comprises the sequences represented by SEQ ID NO: 63 and SEQ ID NO: 64;
a thirty-third primer pair comprises the sequences represented by SEQ ID NO: 65 and SEQ ID NO: 66;
a thirty-fourth primer pair comprises the sequences represented by SEQ ID NO: 67 and SEQ ID NO: 68;
a thirty-fifth pair comprises the sequences represented by SEQ ID NO: 69 and SEQ ID NO: 70; and
a twenty-sixth primer pair comprises the sequences represented by SEQ ID NO: 71 and SEQ ID NO: 72.

2. The kit as claimed in claim 1, wherein each of the microsatellite primer pair is a set of the primer pairs, and the set of the primer pairs comprise: any one nucleotide sequence selected from the group consisting of the first primer pair to the thirty-sixth primer pair.

3. The kit as claimed in claim 1, wherein each of the microsatellite primer pair is a set of the primer pairs, and the set of the primer pairs are selected from the group consisting of the first primer pair to the thirty-sixth primer pair.

4. The kit as claimed in claim 1, wherein the at least one microsatellite primer pair comprises: a forward microsatellite primer and a reverse microsatellite primer.

5. The kit as claimed in claim 4, wherein the 5' end of the forward microsatellite primer is labeled with a fluorescent reagent.

6. The kit as claimed in claim 5, wherein the fluorescent reagent is HEX, FAM, or NED.

7. The kit as claimed in claim 4, wherein the 5' end of the reverse microsatellite primer is labeled with a fluorescent reagent.

8. The kit as claimed in claim 7, wherein the fluorescent reagent is HEX, FAM, or NED.

9. A method for identifying Phalaenopsis varieties, comprising the following steps:
(A) providing a tissue sample of a tested Phalaenopsis variety, and isolating the genomic DNA from the tissue sample;
(B) detecting the genotype of the tissue sample of the tested Phalaenopsis variety by using at least one microsatellite primer pair to obtain a genotype, wherein each of the primer pair comprises: a nucleotide with 75-100% identity to the sequences represented by a primer pair selected from the following groups:
a first primer pair comprises the sequences represented by SEQ ID NO: 1 and SEQ ID NO: 2;
a second primer pair comprises the sequences represented by SEQ ID NO: 3 and SEQ ID NO: 4;
a third primer pair comprises the sequences represented by SEQ ID NO: 5 and SEQ ID NO: 6;
a fourth primer pair comprises the sequences represented by SEQ ID NO: 7 and SEQ ID NO: 8;
a fifth primer pair comprises the sequences represented by SEQ ID NO: 9 and SEQ ID NO: 10;
a sixth primer pair comprises the sequences represented by SEQ ID NO: 11 and SEQ ID NO: 12;
a seventh primer pair comprises the sequences represented by SEQ ID NO: 13 and SEQ ID NO: 14;
an eighth primer pair comprises the sequences represented by SEQ ID NO: 15 and SEQ ID NO: 16;
a ninth primer pair comprises the sequences represented by SEQ ID NO: 17 and SEQ ID NO: 18;
a tenth primer pair comprises the sequences represented by SEQ ID NO: 19 and SEQ ID NO: 20;
an eleventh primer pair comprises the sequences represented by SEQ ID NO: 21 and SEQ ID NO: 22;
a twelfth primer pair comprises the sequences represented by SEQ ID NO: 23 and SEQ ID NO: 24;
a thirteenth primer pair comprises the sequences represented by SEQ ID NO: 25 and SEQ ID NO: 26;
a fourteenth primer pair comprises the sequences represented by SEQ ID NO: 27 and SEQ ID NO: 28;
a fifteenth primer pair comprises the sequences represented by SEQ ID NO: 29 and SEQ ID NO: 30;
a sixteenth primer pair comprises the sequences represented by SEQ ID NO: 31 and SEQ ID NO: 32;
a seventeenth primer pair comprises the sequences represented by SEQ ID NO: 33 and SEQ ID NO: 34;
an eighteenth primer pair comprises the sequences represented by SEQ ID NO: 35 and SEQ ID NO: 36;
a nineteenth primer pair comprises the sequences represented by SEQ ID NO: 37 and SEQ ID NO: 38;
a twentieth primer pair comprises the sequences represented by SEQ ID NO: 39 and SEQ ID NO: 40;
a twenty-first primer pair comprises the sequences represented by SEQ ID NO: 41 and SEQ ID NO: 42;
a twenty-second primer pair comprises the sequences represented by SEQ ID NO: 43 and SEQ ID NO: 44;
a twenty-third primer pair comprises the sequences represented by SEQ ID NO: 45 and SEQ ID NO: 46;
a twenty-fourth primer pair comprises the sequences represented by SEQ ID NO: 47 and SEQ ID NO: 48;
a twenty-fifth primer pair comprises the sequences represented by SEQ ID NO: 49 and SEQ ID NO: 50;
a twenty-sixth primer pair comprises the sequences represented by SEQ ID NO: 51 and SEQ ID NO: 52;
a twenty-seventh primer pair comprises the sequences represented by SEQ ID NO: 53 and SEQ ID NO: 54;
a twenty-eighth primer pair comprises the sequences represented by SEQ ID NO: 55 and SEQ ID NO: 56;
a twenty-ninth primer pair comprises the sequences represented by SEQ ID NO: 57 and SEQ ID NO: 58;
a thirtieth primer pair comprises the sequences represented by SEQ ID NO: 59 and SEQ ID NO: 60;
a thirty-first primer pair comprises the sequences represented by SEQ ID NO: 61 and SEQ ID NO: 62;
a thirty-second primer pair comprises the sequences represented by SEQ ID NO: 63 and SEQ ID NO: 64;
a thirty-third primer pair comprises the sequences respresented by SEQ ID NO: 65 and SEQ ID NO: 66;
a thirty-fourth primer pair comprises the sequences respresented by SEQ ID NO: 67 and SEQ ID NO: 68;
a thirty-fifth pair comprises the sequences respresented by SEQ ID NO: 69 and SEQ ID NO: 70;
a thirty-sixth primer pair comprises the sequences respresented by SEQ ID NO: 71 and SEQ ID NO: 72; and
(C) comparing the genotype obtained from the tested Phalaenopsis variety with a known database of the Phalaenopsis genotype to identify the the tested Phalaenopsis variety.

10. The method as claimed in claim 9, wherein each of the microsatellite primer pair is a set of the primer pair sequences, the primer pair sequences comprise: any one nucleotide sequence selected from the group consisting of the first primer pair to the thirty-sixth primer pair.

11. The method as claimed in claim 9, wherein each of the microsatellite primer pair is a sequence set of the primer pair, the primer pair sequences are selected from the group consisting of the first primer pair to the thirty-sixth primer pair.

12. The method as claimed in claim 9, wherein at least one microsatellite primer pair is used to detect the genomic DNA of the tissue sample via a polymerase chain reaction (PCR).

13. The method as claimed in claim 9, wherein the at least one microsatellite primer pair comprises: a forward microsatellite primer and a reverse microsatellite primer.

14. The method as claimed in claim 13, wherein the 5' end of the forward microsatellite primer is labeled with a fluorescent reagent.

15. The method as claimed in claim 14, wherein the fluorescent reagent is HEX, FAM, or NED.

16. The method as claimed in claim 13, wherein the 5' end of the reverse microsatellite primer is labeled with a fluorescent reagent.

17. The method as claimed in claim 16, wherein the fluorescent reagent is HEX, FAM, or NED.
